# EUROPÄISCHE PATENTSCHRIFT

(11) **EP 3 938 088 B1**
(45) Veröffentlichungstag und Bekanntmachung des Hinweises auf die Patenterteilung: **03.08.2022**
(21) Anmeldenummer: 20731380.0
(22) Anmeldetag: 13.05.2020
(51) Int. Cl.: B01J 3/00, B01J 19/00

(54) **VERWENDUNG EINES MIKROJET-REAKTORS ZUR BEARBEITUNG VON BIOMASSE**
USE OF A MICROJET REACTOR FOR PROCESSING BIOMASS
UTILISATION D'UN RÉACTEUR À MICROJET POUR LE TRAITEMENT DE LA BIOMASSE

(30) Priorität: 13.05.2019 DE 102019112382
(43) Veröffentlichungstag der Anmeldung: 19.01.2022
(73) Patentinhaber: MyBiotech GmbH, 66802 Überherrn (DE)
(72) Erfinder: BAUMSTÜMMLER, Bernd, 66740 Saarlouis (DE); TÜRELI, Akif Emre, 66802 Überherrn (DE); PENTH, Felix, 66822 Lebach (DE); MÜLLER, Daniel, 66802 Überherrn (DE); KRUMBHOLZ, Rudolf, 66589 Merchweiler (DE)
(74) Vertreter: Patentanwaltskanzlei Vièl & Wieske PartGmbB
(86) Internationale Anmeldenummer: PCT/DE2020/100408
(87) Internationale Veröffentlichungsnummer: WO 2020/228908

(56) Entgegenhaltungen:
- WO-A2-00/61275
- DE-A1-102009 008 478
- DE-A1-102016 101 232
- DE-A1-102017 110 292
- DE-A1-102017 110 293
- US-A1- 2016 051 956
- Akif Akif Emre Türeli: "Nanoparticle Preparation Process Using Novel Microjet Reactor Technology for Enhancing Dissolution Rates of Poorly Water Soluble Drugs", , 29. April 2015 (2015-04-29), XP055715779, Gefunden im Internet: URL:https://publications.ub.uni-mainz.de/t heses/volltexte/2015/4045/pdf/4045.pdf [gefunden am 2020-07-16]
- Pharmaceutical Technology Editors: "Nanoparticle Reactor Technology Controls Particle Size and Distribution | Pharmaceutical Technology", , 18. Dezember 2017 (2017-12-18), XP055716054, Gefunden im Internet: URL:http://www.pharmtech.com/nanoparticle- reactor-technology-controls-particle-size- and-distribution [gefunden am 2020-07-17]

## Beschreibung

Die Erfindung betrifft die Verwendung eines Mikrojet-Reaktors zum Zellaufschluss von fließfähiger Biomasse.

Eine Vielzahl biologischer, biotechnologischer, biopharmazeutischer und in zunehmendem Maße auch andere industrielle Verfahren sind heute auf die Produktion von nutzbaren Wertstoffen durch eine Vielfalt an unterschiedlichsten Mikroorganismen, pflanzlichen oder tierischen Geweben angewiesen. Prominentes Beispiel ist die Produktion von Insulin und Insulinanaloga aus gentechnisch veränderten Mikroorganismen. Mit dem fortschreitenden Klimawandel und der inhärenten Begrenztheit fossiler Brennstoffe rücken jedoch beispielsweise auch prinzipiell regenerative Brennstoffe in den Fokus der Forschung und Industrie, der sich zum größten Teil auf die Lipidproduktion aus Mikroalgen richtet.

Eine zunehmend gesundheitsbewusster lebende Bevölkerung lässt den Bedarf an Nahrungsergänzungsmitteln wie den Omega-3-Fettsäuren, darunter vor allem EPA (Eicosapentaensäure) und DHA (Docosahexaensäure) stetig steigen. Der allergrößte Teil dieses Bedarfs wird derzeit jedoch durch den Einsatz von Fischöl gedeckt, wobei an der Nachhaltigkeit des Fischfanges zur Fischölgewinnung durchaus gezweifelt werden kann. Auch für vegetarisch oder vegan lebende Bevölkerungsgruppen ist die Zufuhr von Omega-3-Fettsäuren aus tierischen Quellen problematisch. Daher ist das Interesse an Omega-3-Fettsäuren aus anderen Quellen groß, insbesondere an der Gewinnung von Omega-3-Fettsäuren aus der Produktion von Mikroorganismen wie Mikroalgen, Cyanobakterien oder Hefen.

In den einzelnen Mikroorganismen liegen die Omega-3-Fettsäuren oft jedoch nicht frei vor, auch werden sie nicht in das Zuchtmedium abgegeben. Vielmehr sind sie oft in Form verschiedener Lipide wie Triglyceride, Phospholipide, Galacto- oder Glycolipide gebunden und sind Bestandteile der Zellmembran oder anderer Zellorganellen. Auch in Form von Lipidvesikeln können die Fettsäuren Bestandteile der Zellen sein. Allen diesen Erscheinungsformen ist gemein, dass die Extraktion der Lipide durch die gebundene Form oder den Einschluss innerhalb der Zelle und in den Zellorganellen erschwert wird.

Besonderes Interesse liegt damit auf dem Zellaufschluss und damit der Zerstörung oder Beschädigung der Zellwände und Zellmembranen von fließfähig gemachten Einzelzellen oder Zellverbänden unterschiedlicher Natur, um Zugang zu den in der Zelle enthaltenen Wertstoffen zu erhalten. Zerstörung heißt in diesem Zusammenhang, dass die Zellmembran oder Zellwand soweit beschädigt wird, dass das Zellinnere durch Öffnung von Teilen der Zellmembran oder Zellwand soweit freigelegt wird, dass der Stoffaustausch mit der Umgebung ungehindert oder nahezu ungehindert erfolgen kann, oder eine Abgrenzung von Zellinnerem zur Umgebung nicht mehr eindeutig erfolgen kann. Eine Beschädigung der Zellwand heißt in diesem Zusammenhang, dass die Zellwand oder Zellmembran in ihrer Struktur soweit beschädigt wird, dass durch Risse oder Löcher in der Zellwand oder Zellmembran ein Stoffaustausch zwischen Zellinnerem und der Umgebung an den zelleigenen Transportmechanismen vorbei erfolgen kann. Diese Definition lässt sich in gleicher Weise auch auf die Zellorganellen der Zellen anwenden, die ebenfalls durch Zellmembranen geschützt werden.

Der mikrobielle Zellaufschluss wird nach derzeitigem Stand der Technik durch verschiedene Verfahren gelöst. Dem Fachmann ist hierzu beispielsweise ein Verfahren bekannt, bei dem mittels Eintauchens einer Ultraschall-Sonotrode im Batch-Verfahren kleine Volumina zuverlässig aufgeschlossen werden können. Es existieren daneben auch Durchlaufvarianten, bei denen die Sonotrode in eine geschlossene, durchströmte Kammer eintaucht. Nachteilig hierbei ist neben der schlechten Skalierbarkeit durch ein hohes Investitionsvolumen auch der hohe Sonotrodenverschleiß, wobei sich der metallische Abrieb der Sonotroden zwangsläufig im Produkt wiederfindet. Bei diesem Verfahren treten neben der hohen Lärmbelastung zudem oft lokale Überhitzungen auf, die bei temperaturempfindlichen Proben für eine Reduktion der Ausbeute verantwortlich sein können. Daneben existiert auch das Verfahren des so genannten "Bead-Milling", bei dem der Zellaufschluss in einem Aufschlussbehälter durch Zugabe von Mahlkörpern und Agitation der Mahlkörper erfolgt. Nachteilig hierbei sind die schlechte Skalierbarkeit des Verfahrens sowie die Verfügbarkeit für nur kleine Probenvolumina.

Ebenfalls ist das Verfahren der so genannten "French-Press" bekannt, wonach ein diskretes Probenvolumen durch einen Kolben auf hohen Druck gebracht und anschließend durch einen engen Spalt in einem Ventil gepresst wird. Die Lyse der Zellen erfolgt hierbei durch die auftretenden hohen Scherkräfte. Nachteilig ist hier vor allem die schlechte Skalierbarkeit.

Des Weiteren ist das Verfahren der Hochdruckhomogenisation bekannt, bei dem eine Zellsuspension mit hohem Druck durch einen Ringspalt gepresst wird. Durch Scherkräfte und durch die mögliche Kavitation durch die rasche Entspannung im Spalt, sowie Aufprallkräfte bei Kontakt mit einem den Ringspalt umgebenden Prallring, können im Hochdruckhomogenisator auch Zellen zerstört oder beschädigt werden. Nachteilig bei Verwendung des Hochdruckhomogenisators ist die Notwendigkeit von Minimaldrücken im Bereich von 700 - 800 bar, was den Einsatz teurer Hochdruckkomponenten und ein entsprechendes Investitionsvolumen voraussetzt. Ein weiterer Nachteil des Verfahrens ist der auftretende Verschleiß an den meist metallischen Komponenten des Homogenisators, die durch die Kavitation und die Scherung stark beansprucht werden.

Ein MikroJet-Reaktor ist aus der Patentschrift EP 1 165 224 B1 bekannt.

Die DE 101 41 054 A1 beschreibt einen Strahlreaktor zur Durchführung physikalischer und chemischer Stoffumwandlungen in einem in einem Gasraum liegenden Kollisionspunkt von Flüssigkeitsstrahlen, die Justierung des Reaktors, den Schutz des Reaktors vor Zerstörung durch Kavitation mittels tetraedrisch angeordneter keramischer Kugeln und die Verwendung des Reaktors zum Homogenisieren, Dispergieren und Emulgieren.

Die Dissertation "Nanoparticle preparation Using Novel Microjet Reactor Technology for Enhancing Dissolution Rates of Poorly Water Soluble Drugs" von Akif Emre Türeli beschreibt die Verwendung von MikroJet-Reaktor-Technologie für die spezifische Herstellung von Nanopartikeln. Insbesondere beschreibt die Dissertation die Herstellung magensaftresistenter Nanopartikel der Modellsubstanz Fenofibrat zur oralen Applikation unter Verwendung von Hydroxypropyl-Methylcellulosephtalat und niedermolekularem Chitosan als Matrixsubstanzen.

Die DE 10 2017 110 293 A1 betrifft ein Verfahren zur Oberflächenmodifikation von verkapselten Stoffen. Dabei werden in einem MikroJet-Reaktor mindestens zwei Flüssigkeitsströme einerseits eines mit einem Verkapselungsmaterial verkapselten Stoffes und andererseits eines Oberflächenmodifikators zur Kollision gebracht.

Die US 2016/051956 A1 betrifft eine Vorrichtung und ein Verfahren zur Herstellung von Dispersionen und Feststoffen durch gesteuerte Fällungs-, Mitfällungs- und Selbstorganisationsprozesse in einem MikroJet-Reaktor, wobei ein Lösungsmittelstrahl, der mindestens ein Zielmolekül enthält, und ein Nichtlösungsmittelstrahl miteinander kollidieren.

Die DE 10 2017 110 292 A1 beschreibt ein Verfahren und eine Vorrichtung zum Herstellen von Reaktionsprodukten durch kontrollierte Fällung, Co-Fällung und/oder Selbstorganisationsprozesse in einem MikroJet-Reaktor, wobei jeweils ein aus einer ersten Düse austretender Strahl eines ersten Edukts und ein aus einer zweiten Düse austretender Strahl eines zweiten Edukts mit definierten Drücken und Flussraten im Reaktorraum des Mikrojetreaktors an einem Kollisionspunkt aufeinandertreffen, wobei der Mikrojetreaktor mindestens einen Gaseintritt zum Begasen des Reaktorraumes und einen Produktaustritt zum Austragen der Produkte in einem Gasstrom aufweist.

Die DE 10 2016 101232 A1 beschreibt ein Verfahren zur Herstellung von Emulsionen. Dabei werden mindestens zwei Flüssigkeitsströme von miteinander nicht mischbaren Flüssigkeiten durch getrennte Öffnungen mit definiertem Durchmesser gepumpt, um Fließgeschwindigkeit der Flüssigkeitsströme von mehr als 10 m/s zu erreichen, wobei die Flüssigkeitsströme an einem Kollisionspunkt in einem Raum aufeinandertreffen.

Die DE 10 2009 008 478 A1 betrifft die Herstellung pharmazeutischer Wirkstoffpartikel mit geringer Partikelgröße durch eine Kombination einer Solvent-Nonsolvent-Fällung mit einer Insitu-Sprühtrocknung.

Der Erfindung liegt die Aufgabe zugrunde, den Zellaufschluss von fließfähiger Biomasse und/oder die Extraktion von Bestandteilen daraus zu optimieren.

Dies wird erfindungsgemäß mit Hilfe der Verwendung eines MikroJet-Reaktors gelöst, dass die Düsendurchmesser des MikroJet-Reaktors im Bereich 50 µm - 2000 µm liegen und die hydraulischen Düsenvordrücke des MikroJet-Reaktors im Bereich von 5-1000 bar liegen, der Strahl durch zwei kreisförmige Blenden oder Düsen gebildet wird, die sich an gegenüberliegenden Stellen eines größeren Raumes befinden und der Kollisionswinkel der Stahlen 90° bis 180° beträgt, wobei mehrere Flüssigkeitsstrahlen, die mit Biomasse versetzt sind, mit Geschwindigkeiten zwischen 31 m/s und 447 m/s kollidieren, wobei mindestens einer der mehreren aufeinanderprallenden Flüssigkeitsstrahlen mit intakter oder ganz oder teilweise aufgeschlossener Biomasse versetzt wird, wobei mindestens einer der Strahlen ganz oder teilweise mit einem Extraktionsmittel angereichert wird und zeitgleich mit der Kollision der Flüssigkeitsstrahlen oder im Anschluss daran eine Extraktion erfolgt.

Erfindungsgemäß werden dabei die Zellen in der Biomasse durch die entstehenden Kräfte bei Beschleunigung, Einleitung der Beschleunigung, Aufprall der Strahlen und Mischung der Strahlbestandteile ganz oder teilweise zerstört.

Biomasse meint in diesem Zusammenhang im Allgemeinen flüssige, verflüssigte oder fließfähige oder fließfähig gemachte reine oder verdünnte oder verunreinigte Stoffe, wobei diese Stoffe zum einen aus Einzelzellen oder Zellverbänden bestehen können, zum anderen können diese Stoffe aus mechanisch oder anderweitig vorzerkleinerten oder ganz oder teilweise aufgeschlossenen Zellen oder Zellverbänden bestehen oder von diesen Zellen abgegebene Stoffwechselprodukte sein. Außerdem sind mit Biomasse ebenfalls Extrakte oder Konzentrate aus diesen Stoffen gemeint, beispielsweise Aminosäuren, Fettsäuren, Kohlenhydrate, Extremolyte, Stoffwechselprodukte der Zellen, oder andere Zellinhalte oder Bestandteile daraus oder Bestandteile der Zellwände oder Zellmembranen oder Mischungen aus mindestens zwei der vorgenannten Stoffe. Ebenfalls mit Biomasse gemeint sind komplexe Verbindungen aus und mit den vorgenannten Stoffen, beispielsweise Gemenge enthaltend mindestens einen der nachfolgenden Stoffe: hoch- und niedermolekulare Verbindungen, Vitamine, Pigmente, Bioemulgatoren und Biotenside, Biopolymere wie Proteine und Enzyme, Peptide, Lipide, DNA, RNA, Polysaccharide und Lignin.

Die Zellen sind beispielsweise Mikroalgen, Bakterien, Hefen, oder Zellen pflanzlichen, tierischen oder menschlichen Ursprungs.

Die Zellen werden zunächst in eine fließfähige Form gebracht, beispielsweise als Zellsuspension, oder das verdünnte oder unverdünnte Kulturmedium wird direkt für die weiteren Verfahrensschritte verwendet. Anschließend wird die nun fließfähige Biomasse beschleunigt und in mehreren Flüssigkeitsstrahlen gebündelt. Die Strahlführung ist dabei so gestaltet, dass die Strahlen frontal oder unter einem stumpfen Winkel aufeinanderprallen.

Dabei kommen in dieser einfachen Ausführung der Erfindung mehrere Effekte zur Zerstörung der Zellen zum Tragen. Durch die Beschleunigung des Flüssigkeitsstrahls wird in großem Umfang kinetische Energie für den Zellaufschluss bereitgestellt. Die nach der Beschleunigung anschließende Kollision der Strahlen mit der Umwandlung dieser Energie in mechanische Arbeit sorgt für das Auftreten von Druck- und Scherkräften, die auch auf die Zellen wirken. In einer bevorzugten Ausführung der Erfindung wird die Beschleunigung durch einen hohen Vordruck einer Düsenströmung erzeugt, wodurch zum anderen auch am Düseneinlass Scherkräfte an den Zellen wirksam werden, da dort eine starke Querschnittsreduzierung der Strömung eine hohe Geschwindigkeit um eine Scherkante erzwingt. Diese Effekte bewirken einen Aufschluss der Zelle durch Zerstörung der Zellwand.

Diese Verwendung dient der Extraktion von Bestandteilen der Biomasse. Diese Extraktion erfolgt zeitgleich mit der Kollision der Flüssigkeitsstrahlen oder im Anschluss daran.

Extraktion in diesem Zusammenhang meint physikalische oder chemische Vorgänge, die zur An- oder Abreicherung eines Zielproduktes oder einer Verunreinigung in einem Stoffgemenge oder einem Lösungsmittel oder zur Abtrennung eines Zielproduktes oder Verunreinigung aus einem Stoffgemenge oder einem Lösungsmittel geeignet sind, oder eine Kombination daraus. Konkrete Verfahren der Extraktion können beispielsweise Flüssig-Flüssig-Extraktion, Fest-Flüssig-Extraktion, Flüssig-Gas-Extraktion, Gas-Flüssig-Extraktion, Extraktion mit überkritischen Fluiden, Kristallisation, Destillation, Wasserdampfdestillation, Filtration, Permeation, Pervaporation, Elektrophorese, Fällung, Flotation, Flockung, Sedimentation, Zentrifugation oder Chromatografie oder weitere dem Fachmann bekannte Verfahren sein. Außerdem sind mit Extraktion Verfahren gemeint, die mittels einer chemischen Reaktion Bestandteile der Biomasse aus einer gebundenen Form freisetzen oder in eine andere gebundene Form überführen, oder Bestandteile der Biomasse mittels einer chemischen Reaktion umwandeln. Des Weiteren sind mit Extraktion Verfahren gemeint, die mehrere der vorgenannten Verfahren parallel oder hintereinander kombinieren.

Hierzu kann vorgesehen sein, dass mindestens einer der Strahlen ganz oder teilweise mit einem Extraktionsmittel angereichert wird.

Eine weitere bevorzugte Verwendung besteht darin, dass das Gehäuse gasgefüllt oder gasdurchströmt ist. Somit findet die Kollision in einer Gasatmosphäre statt. Die Gasatmosphäre kann einerseits durch die räumliche Gestaltung des Kollisionsortes der Strahlen geschehen, indem der Kollision genügend Raum gegeben wird und sich die Strahlen frei ausbilden können. Andererseits kann der Kollisionsort auch von einem Gas durchströmt werden und so das Kollisionsprodukt vom Gasstrom abtransportiert werden.

Eine weitere Variante der Verwendung besteht darin, dass die Zerstörung der Zellen durch Zugabe von Hilfsstoffen in fließfähige Biomasse initiiert oder begünstigt wird. Dabei können die Hilfsstoffe und die fließfähige Biomasse sich in getrennten Flüssigkeitsstrahlen befinden. Des Weiteren kann der Hilfsstoff ein Enzym, ein Salz, ein organisches Lösungsmittel, eine Säure oder eine Lauge sein. Auch eine Mischung aus mindestens zwei dieser Substanzen ist denkbar.

Eine weitere mögliche Verwendung beinhaltet, dass in mindestens einen Flüssigkeitsstrahl vor der Strahlbildung ein Gas oder Flüssiggas eingebracht wird. Dabei wird den Flüssigkeitsstrahlen vor der Beschleunigung ein Gas oder ein Flüssiggas zugesetzt. Dieses Gas hat die Aufgabe, sich bei hohem Druck in den Zellen zu lösen und beim Verlassen der Beschleunigungsstrecke durch den entstehenden Druckabfall zu expandieren und die Zellen von innen heraus zu zerstören oder die Zellwand oder Zellmembran zu perforieren.

In einer weiteren erfindungsgemäßen Verwendung werden die Flüssigkeitsstrahlen vor der Beschleunigung auf eine Temperatur über dem Normaldrucksiedepunkt der jeweiligen Flüssigkeit, vorzugsweise Wasser, erhitzt. Da die Zellen zu einem großen Teil aus Wasser bestehen, wird ein Teil des Wassers beim Verlassen der Beschleunigungsstrecke durch den entstehenden Druckabfall verdampfen. Der entstehende Wasserdampf innerhalb der Zellen hat die Aufgabe, diese von innen heraus zu zerstören oder die Zellwand oder Zellmembran zu perforieren.

In einer weiteren Ausführung der Erfindung werden klassischerweise nachgeschaltete Reaktions- und Extraktionsschritte in das Aufschlussverfahren integriert. Nutzt man beispielsweise eines der erfindungsgemäßen Verfahren zum Zellaufschluss von Mikroalgen zur Gewinnung von Omega-3-Fettsäuren, so ist es unter anderem durch Zugabe von einem Alkohol, einem Umesterungskatalysator und der Bereitstellung von Reaktionswärme möglich, die in Lipiden gebundenen Fettsäuren mittels Veresterung von der Kopfgruppe zu lösen und die entsprechenden Ester zu bilden. Auch andere Formen der Veresterung der Fettsäurereste der Lipide sind denkbar, in denen beispielsweise der Alkohol als Alkoholat zugesetzt wird. Die Veresterung wird erfindungsgemäß dadurch realisiert, dass die zur Veresterung nötigen Komponenten in die fließfähige Biomasse eingemischt werden. Dies kann erfindungsgemäß vor Beginn des eigentlichen Aufschlussprozesses geschehen oder effektiverweise bevorzugt durch die Kollision als Hochgeschwindigkeitsstrahl mit einem Flüssigkeitsstrahl der Zellsuspension. Die hervorragende Mischung durch die Strahlkollision begünstigt und beschleunigt die Veresterung, so dass in geringerer Zeit eine höhere Ausbeute im Vergleich mit konventionellen Mischverfahren erreicht werden kann.

In gleicher Weise kann ein Extraktionsprozess erfolgen, in dem die nun veresterten Fettsäuren als Hochgeschwindigkeitsstrahl mit einem Flüssigkeitsstrahl des lipophilen Extraktionsmittels zur Kollision gebracht werden. Die hervorragende Mischung durch die Strahlkollision kann die klassischen, oft aufwändig gestalteten Kolonnen zur Flüssig-Flüssig-Extraktion vollständig ersetzen.

Diese beispielhaft genannten Reaktions- und Extraktionsprozesse können sowohl gleichzeitig als auch nach dem Zellaufschluss erfolgen, in vielen Fällen jedoch sogar innerhalb derselben Vorrichtung. Auch ist es in einer weiteren Ausführung der Erfindung möglich, die Reaktionsmittel nach dem Aufschlussprozess klassisch mit der aufgeschlossenen fließfähigen Biomasse zu vermischen und durch das erfindungsgemäße Verfahren eine vollständige Durchmischung zu erreichen. Ebenso ist es möglich, die Extraktionsmittel nach dem Reaktionsprozess, soweit notwendig, oder eben entsprechend nach dem Aufschlussprozess klassisch mit der aufgeschlossenen fließfähigen Biomasse zu vermischen und durch das erfindungsgemäße Verfahren eine vollständige Durchmischung zu erreichen.

Die Aufgabe wird mit einer Vorrichtung zur Bearbeitung der Biomasse dadurch gelöst, dass die Vorrichtung ein MikroJet-Reaktor ist und dass ein Prallkörper, vorzugsweise aus Keramik, Glas oder einem Metall in den Kollisionspunkt bringbar ist. Durch die Kollision der Flüssigkeitsstrahlen mit einer Prallplatte aus einem festen Material oder mit einen in die Vorrichtung eingebrachten Prallkörper aus einem festen Material wird vorteilhaft der Zellaufschluss von fließfähiger Biomasse durch die Zerstörung der Zellwände bei der Wirkung der mechanischen Kräfte beim Aufprallen auf den Prallkörper verbessert.

Eine Weiterbildung der Erfindung besteht darin, dass mindestens eine Pumpe durch einen flüssigkeitsgefüllten Zylinder gebildet wird, der mittels eines komprimierten Gases auf Druck zwischen 5 und 1000 bar bringbar ist. Dies ermöglicht einen höheren Druck und somit eine höhere Geschwindigkeit der kollidierenden Flüssigkeitsstrahlen, was die Zellaufspaltung vorteilhaft verbessert.

Hierbei ist es zweckmäßig, dass die Öffnungen im Gehäuse Spalte oder Ringspalte sind.

Allen erfindungsgemäßen Vorrichtungen gemein ist die Notwendigkeit der Beschleunigung der Biomasse und die Bildung von Strahlen, die zur Kollision gebracht werden können. Die Beschleunigung kann ganz allgemein dadurch geschehen, dass die Biomasse unter Druck gesetzt und durch eine Querschnittsreduzierung auf einen niedrigeren Druck entspannt wird, wobei die Strömungsquerschnitte geometrisch so angeordnet werden, dass die sich bildenden Strömungen kollidieren.

In einer bevorzugten Ausführung der Erfindung, im folgenden RUPEX (Rupturing Extractor) genannt, wird der Strahl durch zwei kreisförmige Blenden oder Düsen gebildet, die sich an gegenüberliegenden Stellen eines größeren Raumes befinden. Der Kollisionswinkel der Strahlen beträgt dabei bevorzugt 90°-180°, bevorzugt 135°-180° und besonders bevorzugt 170°-180°. Der Kollisionswinkel ist dabei der kleinere der zwei Winkel, die durch die direkte Verbindung der Strahleinlässe in die Kollisionskammer mit dem Kollisionspunkt gebildet werden. Bevorzugte Ausführungen des RUPEX verwenden dabei Düsendurchmesser im Bereich 50 µm-2000 µm, bevorzugt 200 µm - 1500 µm und hydraulische Düsenvordrücke von 5-1000 bar, bevorzugt von 50-800 bar, und besonders bevorzugt von 100-350 bar. Die Strahlgeschwindigkeiten betragen dabei 31 m/s - 447 m/s, bevorzugt 100 m/s - 400 m/s und besonders bevorzugt 140m/s - 254 m/s. Diese geometrische Anordnung lässt die Strahlen in der mittleren Kammer des RUPEX kollidieren. Das Innere der Kammer wird dann während des Prozesses von der injizierten Flüssigkeit gefüllt und bei entsprechend ansteigendem Druck aus der Kammer transportiert.

In einer weiteren, besonders bevorzugten Ausführung der Erfindung wird die mittlere Kammer des RUPEX von Gas durchströmt, welches die injizierte Flüssigkeit aus der Kammer austrägt und somit für eine Verbesserung der Strahlbildung und der Ausformung des Kollisionsbereiches sorgt. Außerdem lässt sich somit die Kollisionsumgebung inertisieren. Der Kammerdruck beträgt hier bevorzugt 0-300 bar(a), mehr bevorzugt 1-150 bar(a) und noch mehr bevorzugt 2-100 bar(a).

In einer weiteren Ausführung der erfindungsgemäßen Vorrichtung besteht mindestens einer der Strahlen aus einem überkritischen Fluid, oder ein überkritisches Fluid ist Bestandteil mindestens einer der Strahlen.

In einer weiteren Ausführung der erfindungsgemäßen Vorrichtung wird die RUPEX-Vorrichtung nicht kontinuierlich betrieben, sondern intermittierend derart, dass jeweils gegenüberliegende Düsen gleichzeitig kurz andauernde Strahlen formen.

Eine beispielhafte Ausführung der Erfindung sieht zunächst die Herstellung einer Suspension aus 10 g getrockneter Spirulina platensis und 190 g Wasser vor. Diese wird mittels einer Hydraulik-Membranpumpe auf einen Druck von 95 bar gebracht. Der Flüssigkeitsstrom wird in zwei Teile geteilt und in eine RUPEX-Vorrichtung mit einer Düsenweite von 200µm injiziert. Das Kollisionsprodukt verlässt die RUPEX-Vorrichtung durch den sich in der Kollisionskammer aufbauenden Druck und wird in den Ansaugbehälter der Pumpe zurückgeführt. Der Aufschlussprozess ist nach einer halben Stunde abgeschlossen.

Ein weiteres Beispiel sieht zunächst die Herstellung einer Corynebacterium glutamicum-Kultur durch Fermentation vor. Ein Liter der im Verhältnis 1:5 mit Wasser verdünnten Fermentationsbrühe wird mittels einer Hydraulik-Membranpumpe auf einen Druck von 95 bar gebracht, der Flüssigkeitsstrom in zwei Teile geteilt und in eine RUPEX-Vorrichtung mit einer Düsenweite von 200µm injiziert. Das Kollisionsprodukt verlässt die RUPEX-Vorrichtung durch den sich in der Kollisionskammer aufbauenden Druck und wird in den Ansaugbehälter der Pumpe zurückgeführt. Der Aufschlussprozess ist nach einer Stunde abgeschlossen.

Ein weiteres Beispiel sieht zunächst die Herstellung einer Saccharomyces cerevisiae-Kultur durch Fermentation vor. Mittels einer Membranpumpe wird die unverdünnte Kultur mittels einer Hydraulik-Membranpumpe auf einen Druck von 95 bar gebracht, der Flüssigkeitsstrom in zwei Teile geteilt und in eine RUPEX-Vorrichtung mit einer Düsenweite von 800 µm injiziert. Das Kollisionsprodukt verlässt die RUPEX-Vorrichtung durch den sich in der Kollisionskammer aufbauenden Druck und wird in den Ansaugbehälter der Pumpe zurückgeführt. Der Aufschlussprozess ist nach 5 Minuten abgeschlossen.

Ein weiteres Beispiel sieht zunächst die Herstellung einer Curcuma longa - Suspension vor, indem zunächst 20g der Wurzel getrocknet, gehäckselt und anschließend in 36g Wasser und 144g Ethanol suspendiert werden. Die Suspension wird in einen Zylinder gefüllt und mittels einer Stickstoff-Gasflasche auf einen Druck von 180 bar gebracht. Die Suspension wird unter Druck durch eine Rohrleitung zu einer RUPEX-Vorrichtung geführt und durch Düsen mit einem Durchmesser von 1500µm injiziert. Neben dem Aufschluss der Zellen erfolgt in diesem Schritt gleichzeitig eine Extraktion. Der Extrakt verlässt die RUPEX-Vorrichtung mit Hilfe eines Inertgases, welches die RUPEX-Vorrichtung durchströmt.

Ein weiteres Beispiel sieht zunächst die Herstellung einer Nannochloropsis oceanica-Kultur durch Kultivierung in einem Photobioreaktor vor. Mittels zweier Plungerpumpen werden 100 Liter der unverdünnten, aufkonzentrierten Kultur auf einen Druck von 320 bar gebracht und in jeweils zwei Rohrleitungen zu zwei RUPEX-Vorrichtungen geführt und durch jeweils zwei gegenüberliegende Düsen mit einem Durchmesser von 1200µm injiziert und im Inneren der RUPEX-Vorrichtungen zur Kollision gebracht. Das Kollisionsprodukt verlässt den Ausgang der RUPEX-Vorrichtung mit Hilfe eines Inertgases, welches die RUPEX-Vorrichtungen durchströmt, und fließt in den Ansaugtank der Pumpen zurück. Der Aufschlussprozess ist nach 10 Minuten abgeschlossen. Die aufgeschlossene Zellsuspension wird mittels einer Kreiselpumpe auf einen Druck von 10 bar gebracht und von einer Seite in eine weitere RUPEX-Vorrichtung mit einer Düsenweite von 300µm injiziert. Als Extraktionsmittel wird n-Hexan mittels einer Kreiselpumpe auf einen Druck von 10 bar gebracht und von der anderen Seite in die RUPEX-Vorrichtung injiziert. Nach komplettem Durchlauf der RUPEX-Vorrichtung kann eine aufschwimmende Phase als Extrakt abgenommen werden.

Ein weiteres Beispiel sieht zunächst die Herstellung einer Suspension aus 11g Nannochloropsis oceanica, 1,3 Liter Methanol, 0,2 Liter Wasser und 0,035 Liter Schwefelsäure vor. Diese Suspension wird mittels einer Hydraulikmembranpumpe auf einen Druck von 95 bar gebracht, durch Rohrleitungen geführt und in einem Wärmetauscher auf 60 °C erhitzt, der Stoffstrom geteilt und von zwei Seiten in eine RUPEX-Vorrichtung mit einer Düsenweite von 200µm injiziert. Das Kollisionsprodukt verlässt den Ausgang der RUPEX-Vorrichtung in einen Tank mit einem Überdruck von 2 bar und wird durch die Pumpe im Kreislauf geführt. Bei diesem Prozess werden der Aufschluss der Zellen und die Veresterung der Nannochloropsis-Lipide zu Methylestern gleichzeitig durchgeführt. Der Prozess ist nach 3 Stunden abgeschlossen. Die Fettsäuremethylester werden im Anschluss extrahiert, indem der Tankinhalt erneut mit einem Druck von 10 bar von einer Seite in die RUPEX-Vorrichtung injiziert wird. Von der anderen Seite wird 0,5 Liter n-Hexan mittels einer Membranpumpe unter einem Druck von 10 bar injiziert. Aus dem Tank kann im Anschluss eine aufschwimmende Phase als Extrakt abgenommen werden.

Nachfolgend werden Ausführungsbeispiele der Erfindung anhand von Zeichnungen näher beschrieben.

### Es zeigen

- Fig. 1: eine erfindungsgemäße Vorrichtung im Querschnitt,
- Fig. 2: eine weitere Ausführung der erfindungsgemäßen Vorrichtung im Querschnitt,
- Fig. 3: eine weitere schematische Repräsentation der erfindungsgemäßen Vorrichtung,
- Fig. 4: eine weitere schematische Repräsentation der erfindungsgemäßen Vorrichtung,
- Fig. 5: eine weitere schematische Repräsentation der erfindungsgemäßen Vorrichtung,
- Fig. 6: eine weitere schematische Repräsentation der erfindungsgemäßen Vorrichtung,
- Fig. 7: eine weitere schematische Repräsentation der erfindungsgemäßen Vorrichtung,
- Fig. 8: eine weitere schematische Repräsentation der erfindungsgemäßen Vorrichtung.

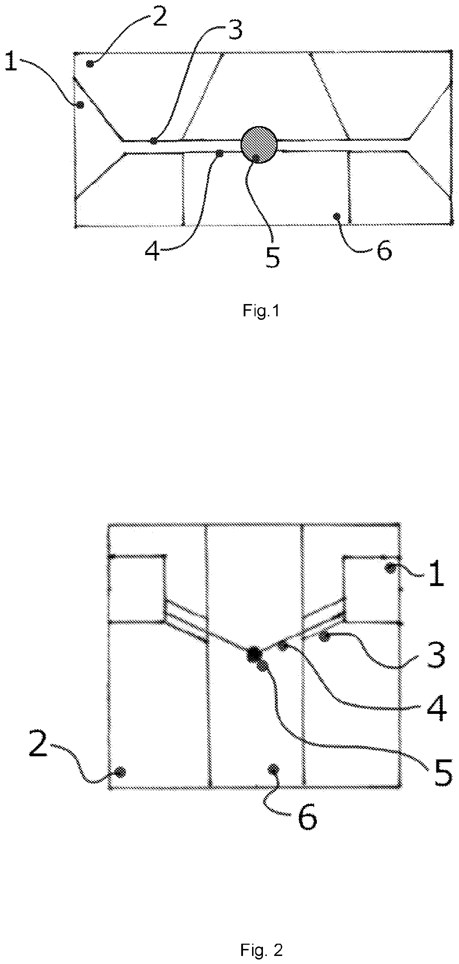

Eine bevorzugte Ausführung der Erfindung ist in Fig. 1 gezeigt. Hierbei ist das Gehäuse der RUPEX-Vorrichtung 2 abgebildet, in welches die fließfähige Biomasse 1 mit hohem Druck von zwei gegenüberliegenden Seiten injiziert und durch eine Düse 3 zu Strahlen 4 geformt wird, welche in der mittleren Kammer (6) der RUPEX-Vorrichtung im Kollisionspunkt 5 kollidieren. In einer weiteren bevorzugten Ausführung der Erfindung kann die mittlere Kammer mit Gas gefüllt oder gasdurchströmt sein, beispielsweise von oben, um den Stoffstrom nach unten auszutragen.

Eine weitere Ausführung der Erfindung ist in Fig. 2 gezeigt. Hierbei ist das Gehäuse der RUPEX-Vorrichtung 2 abgebildet, in welches die fließfähige Biomasse 1 mit hohem Druck von zwei Seiten injiziert und durch zwei Düsen 3, deren Längsachsen in stumpfem Winkel zueinander stehen, zu Strahlen 4 geformt wird, welche in der mittleren Kammer 6 der RUPEX-Vorrichtung unter einem stumpfen Winkel im Kollisionspunkt 5 kollidieren. In einer weiteren, bevorzugten Ausführung der Erfindung kann die mittlere Kammer mit Gas gefüllt oder gasdurchströmt sein, beispielsweise von oben, um den Stoffstrom nach unten auszutragen.

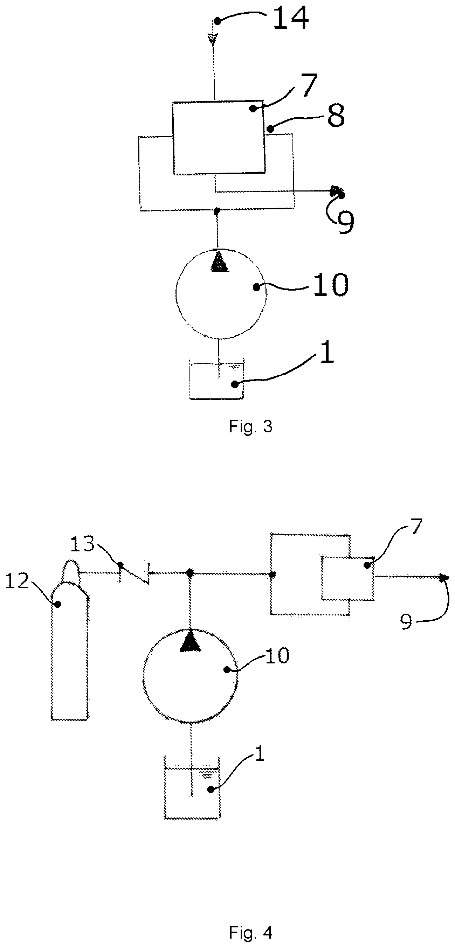

Fig. 3 zeigt eine gerätetechnische Realisierung der RUPEX-Vorrichtung, wobei die fließfähige Biomasse 1 mittels einer Pumpe 10 auf hohen Druck gebracht wird und mittels zweier Düsen 8 in eine Kammer 7 injiziert wird, in der eine Kollision der beiden Flüssigkeitsstrahlen herbeigeführt wird. Ein Gasanschluss 14 sorgt für eine Durchströmung der Kammer mit ebendiesem Gas und trägt das Kollisionsprodukt durch den Ausgang 9 aus.

Eine weitere Ausführung der Erfindung ist in Fig. 4 gezeigt. Hierbei wird der mittels einer Pumpe 10 auf hohen Druck gebrachten, fließfähigen Biomasse 1 ein Gas oder Flüssiggas 12 über ein Ventil 13 zugesetzt. Die mit Gas oder Flüssiggas versetzte Biomasse wird von zwei Seiten in eine RUPEX-Kammer 7 injiziert, in der eine Kollision der Strahlen herbeigeführt wird. Das zugesetzte Gas erzeugt bei der Expansion eine Gasatmosphäre, in der diese Kollision stattfinden kann. Das Gas trägt das Kollisionsprodukt durch den Ausgang 9 aus.

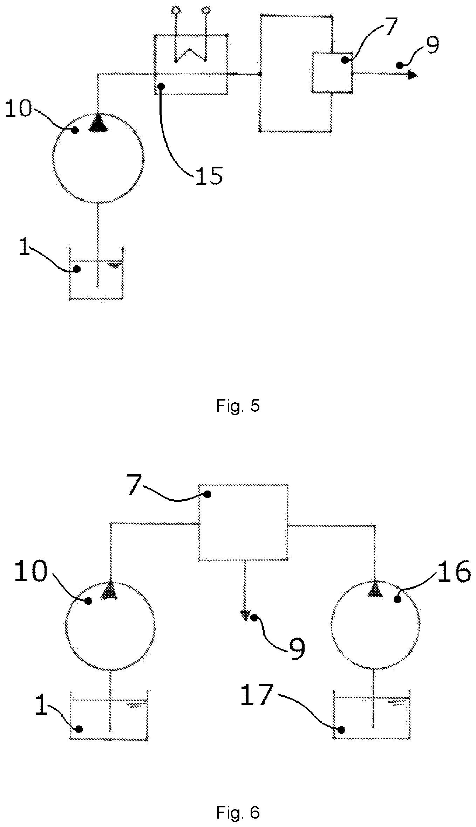

Eine weitere Ausführung der Erfindung ist in Fig. 5 gezeigt. Hierbei wird die mittels einer Pumpe 10 auf hohen Druck gebrachtem, fließfähigen Biomasse 1 mittels eines Wärmetauschers oder einer Heizung 15 auf eine Temperatur über dem Normaldrucksiedepunkt der Trägerflüssigkeit geheizt und von zwei Seiten in eine RUPEX-Kammer 7 injiziert, in der eine Kollision der Strahlen herbeigeführt wird. Die Entspannung der Flüssigkeit in der RUPEX-Kammer auf einen Druck unter ihrem Dampfdruck bewirkt eine mindestens teilweise Verdampfung der Trägerflüssigkeit und einen Produktaustrag aus der RUPEX-Kammer durch den Ausgang 9.

Eine weitere Ausführung der Erfindung ist in Fig. 6 gezeigt. Hierbei wird die mittels einer Pumpe 10 auf hohen Druck gebrachte, fließfähige Biomasse 11 von einer Seite in eine RUPEX-Kammer 7 injiziert. Von der anderen Seite wird eine mittels einer Pumpe 16 auf hohen Druck gebrachte wässrige Lösung eines Enzyms 17 als Aufschlusshilfsstoff injiziert und beide Strahlen in der RUPEX-Kammer 7 zur Kollision gebracht. Das Kollisionsprodukt verlässt die RUPEX-Kammer durch den Ausgang 9.

Eine weitere Ausführung der Erfindung ist mit der Vorrichtung aus Fig. 6 möglich. Hierbei wird die mittels einer Pumpe 10 auf hohen Druck gebrachte, fließfähige Biomasse 1 von einer Seite in eine RUPEX-Kammer 7 injiziert. Von der anderen Seite wird ein mittels einer Pumpe 16 auf hohen Druck gebrachtes Extraktionsmittel 17 injiziert und beide Strahlen in der RUPEX-Kammer 7 zur Kollision gebracht. Das Kollisionsprodukt verlässt die RUPEX-Kammer durch den Ausgang 9.

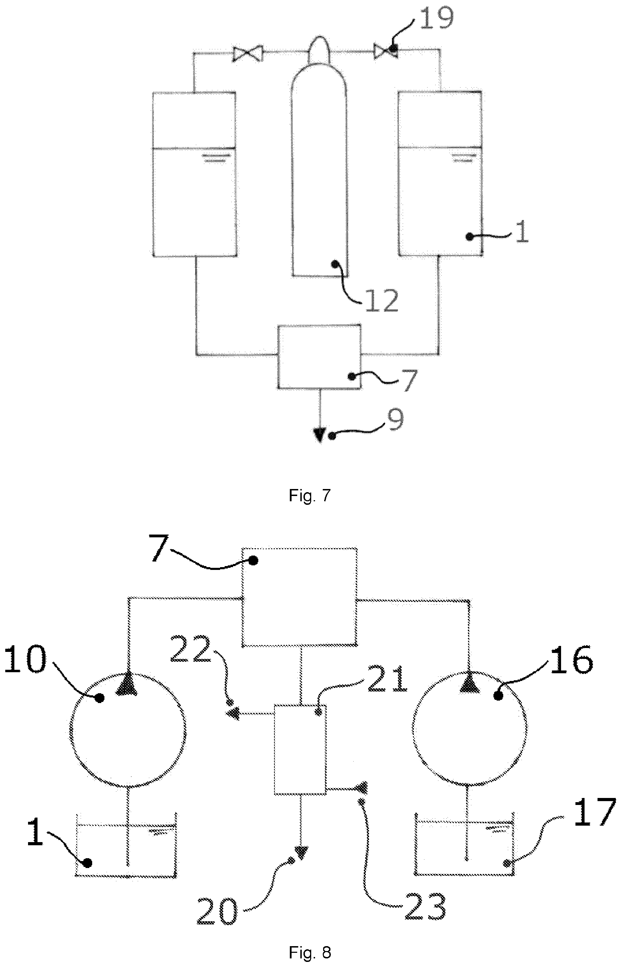

Eine weitere Ausführung der Erfindung zeigt die Vorrichtung in Fig. 7. Hierbei wird die fließfähige Biomasse 1 mittels eines vorkomprimierten Gases oder Flüssiggases 12 und eines Regelventils 19 auf hohen Druck gebracht und von zwei Seiten in die RUPEX-Kammer 7 injiziert und zur Kollision gebracht. Das Kollisionsprodukt verlässt die RUPEX-Kammer durch den Ausgang 9.

Eine weitere Ausführung der Erfindung zeigt die Vorrichtung in Fig. 8. Hierbei wird die mittels einer Pumpe 10 auf hohen Druck gebrachte, fließfähige Biomasse 1 von einer Seite in eine RUPEX-Kammer 7 injiziert. Von der anderen Seite wird ein mittels einer Pumpe 16 auf hohen Druck gebrachtes Extraktionsmittel 17 injiziert und beide Strahlen in der RUPEX-Kammer 7 zur Kollision gebracht. Das Kollisionsprodukt wird anschließend ein eine Extraktionskammer 21 geführt. Das Extraktionsmittel mit Extrakt verlässt die Extraktionskammer durch den Ausgang 22. Über den optionalen Eingang 23 kann zusätzliches Extraktionsmittel oder ein Extraktionshilfsmittel zugeführt werden. Das Raffinat verlässt die Extraktionskammer durch den Ausgang 20.

Eine weitere Ausführung der Erfindung ist mit der Vorrichtung aus Fig. 8 möglich. Hierbei wird die mittels einer Pumpe 10 auf hohen Druck gebrachte, fließfähige Biomasse 1 von einer Seite in eine RUPEX-Kammer 7 injiziert. Von der anderen Seite wird eine mittels einer Pumpe 16 auf hohen Druck gebrachte Biomasse 17 injiziert und beide Strahlen in der RUPEX-Kammer 7 zur Kollision gebracht. Die Pumpe 10 und Pumpe 16 als auch die Biomasse 1 und Biomasse 17 können hierbei identisch sein. Das Kollisionsprodukt wird anschließend in eine Extraktionskammer 21 geführt. Hierin wird ein Extraktionsmittel über den Eingang 23 zugeführt. Das Extraktionsmittel mit Extrakt verlässt die Extraktionskammer über den Ausgang 22. Das Raffinat verlässt die Extraktionskammer durch den Ausgang 20.

## Patentansprüche

1. Verwendung eines MikroJet-Reaktors zum Zellaufschluss von fließfähiger Biomasse (1), **dadurch gekennzeichnet, dass** die Düsendurchmesser des MikroJet-Reaktors im Bereich 50 µm - 2000 µm liegen und die hydraulischen Düsenvordrücke des MikroJet-Reaktors im Bereich von 5-1000 bar liegen, der Strahl durch zwei kreisförmige Blenden oder Düsen gebildet wird, die sich an gegenüberliegenden Stellen eines größeren Raumes befinden und der Kollisionswinkel der Stahlen 90° bis 180° beträgt, wobei mehrere Flüssigkeitsstrahlen (4), die mit Biomasse (1) versetzt sind, mit Geschwindigkeiten zwischen 31 m/s und 447 m/s kollidieren, wobei mindestens einer der mehreren aufeinanderprallenden Flüssigkeitsstrahlen (4) mit intakter oder ganz oder teilweise aufgeschlossener Biomasse versetzt wird, wobei mindestens einer der Strahlen (4) ganz oder teilweise mit einem Extraktionsmittel angereichert wird und zeitgleich mit der Kollision der Flüssigkeitsstrahlen oder im Anschluss daran eine Extraktion erfolgt.

2. Verwendung nach Anspruch 1, **dadurch gekennzeichnet, dass** die Düsendurchmesser des MikroJet-Reaktors im Bereich 200 µm - 1500 µm liegen und die hydraulischen Düsenvordrücke des MikroJet-Reaktors im Bereich von 50-800 bar, und bevorzugt von 100-350 bar liegen und der Kollisionswinkel der Stahlen 135° bis 180° und besonders bevorzugt 170° bis 180° beträgt, wobei mehrere Flüssigkeitsstrahlen (4), die mit Biomasse (1) versetzt sind, mit Geschwindigkeiten zwischen 100 m/s und 400 m/s und besonders zwischen 140m/s und 254 m/s kollidieren.

3. Verwendung nach einem der Ansprüche 1 bis 2, **dadurch gekennzeichnet, dass** die Kollision der Flüssigkeitsstrahlen in einem gasgefüllten oder gasdurchströmten Raum stattfindet.

4. Verwendung nach einem der Ansprüche 1 bis 3, **dadurch gekennzeichnet, dass** die Zerstörung der Zellen durch Zugabe von Hilfsstoffen in fließfähige Biomasse (1) initiiert oder begünstigt wird, wobei die Hilfsstoffe Enzyme, Salze, organische Lösungsmittel, Säuren oder Laugen sind.

5. Verwendung nach einem der Ansprüche 1 bis 4, **dadurch gekennzeichnet, dass** in mindestens einen Flüssigkeitsstrahl (4) vor der Strahlbildung ein Gas oder Flüssiggas (12) eingebracht wird.

6. Verwendung nach einem der Ansprüche 1 bis 5, **dadurch gekennzeichnet, dass** mindestens ein Flüssigkeitsstrahl (4) vor der Strahlbildung auf eine Temperatur über den Normalsiedepunkt der Flüssigkeit gebracht wird.

7. Verwendung nach einem der Ansprüche 1 bis 6, **dadurch gekennzeichnet, dass** zur weiteren Verarbeitung notwendige Reaktionsmittel Bestandteil mindestens eines der Flüssigkeitsstrahlen (4) ist.

8. Verwendung nach Anspruch 7, **dadurch gekennzeichnet, dass** die Reaktionsmittel zur Veresterung von in Lipiden gebundenen oder freien Fettsäuren aus einer Biomasse (1) aus einer Mischung aus einer Säure und einem Alkohol besteht.

9. Verwendung nach Anspruch 7, **dadurch gekennzeichnet, dass** die Reaktionsmittel zur Veresterung von Fettsäuren aus einer Biomasse (1) aus einer Mischung aus einem Alkoholat und dem entsprechenden Alkohol besteht.

10. Verwendung nach Anspruch 7, **dadurch gekennzeichnet, dass** das Reaktionsmittel zur Verseifung von in Lipiden gebundenen oder freien Fettsäuren aus einer Biomasse (1) aus einer Lauge, vorzugsweise Natronlauge oder Kalilauge oder einer Mischung aus Natronlauge und Kalilauge, besteht.

## Claims

1. Use of a microjet reactor for the cell lysis of flowable biomass (1), **characterised in that** the nozzle diameters of the microjet reactor are in the range 50 µm - 2000 µm and the hydraulic nozzle primary pressures of the microjet reactor are in the range of 5-1000 bar, the jet is formed by two circular diaphragms or nozzles which are situated at opposite locations of a larger space, and the collision angle of the jets is 90° to 180°, wherein multiple liquid jets (4), to which biomass (1) has been added, collide at speeds between 31 m/s and 447 m/s, wherein intact or wholly or partially lysed biomass is added to at least one of the multiple colliding liquid jets (4), wherein at least one of the jets (4) is enriched wholly or partially with an extracting agent and an extraction takes place simultaneously with the collision of the liquid jets or subsequently thereto.

2. Use according to claim 1, **characterised in that** the nozzle diameters of the microjet reactor are in the range of 200 µm - 1500 µm and the hydraulic nozzle primary pressures of the microjet reactor are in the range of 50-800 bar, and preferably of 100-350 bar, and the collision angle of the jets is 135° to 180° and particularly preferably 170° to 180°, wherein multiple liquid jets (4), to which biomass (1) has been added, collide at speeds between 100 m/s and 400 m/s and in particular between 140 m/s and 254 m/s.

3. Use according to any one of claims 1 to 2, **characterised in that** the collision of the liquid jets takes place in a space which is filled with gas or is flowed through by gas.

4. Use according to any one of claims 1 to 3, **characterised in that** the destruction of the cells is initiated or facilitated by the addition of auxiliary substances to flowable biomass (1), wherein the auxiliary substances are enzymes, salts, organic solvents, acids or lyes.

5. Use according to any one of claims 1 to 4, **characterised in that** a gas or liquid gas (12) is introduced into at least one liquid jet (4) before the jet is formed.

6. Use according to any one of claims 1 to 5, **characterised in that** at least one liquid jet (4) is brought to a temperature above the normal boiling point of the liquid before the jet is formed.

7. Use according to any one of claims 1 to 6, **characterised in that** reactant necessary for further processing is a constituent of at least one of the liquid jets (4).

8. Use according to claim 7, **characterised in that** the reactant for esterification of lipid-bound or free fatty acids from a biomass (1) consists of a mixture of an acid and an alcohol.

9. Use according to claim 7, **characterised in that** the reactant for esterification of fatty acids from a biomass (1) consists of a mixture of an alcoholate and the corresponding alcohol.

10. Use according to claim 7, **characterised in that** the reactant for hydrolysis of lipid-bound or free fatty acids from a biomass (1) consists of a lye, preferably sodium hydroxide solution or potassium hydroxide solution or a mixture of sodium hydroxide solution and potassium hydroxide solution.

## Revendications

1. Utilisation d'un réacteur à microjets pour la désagrégation cellulaire d'une biomasse capable de s'écouler (1), **caractérisée en ce que** les diamètres de buses du réacteur à microjets se situent dans la plage 50 µm - 2 000 µm, et les pressions hydrauliques d'alimentation des buses du réacteur à microjets se situent dans la plage 5 - 1 000 bars, le jet est formé par deux diaphragmes ou buses circulaires qui se trouvent à des endroits opposés d'un espace étendu et l'angle de collision des jets est compris entre 90° et 180°, plusieurs jets de liquide (4) chargés en biomasse (1) entrant en collision à des vitesses comprises entre 31 m/s et 447 m/s, l'un au moins des plusieurs jets de liquide (4) entrant en collision étant chargé de biomasse intacte, ou totalement ou partiellement désagrégée, au moins l'un des jets (4) étant totalement ou partiellement enrichi d'un agent d'extraction et une extraction étant effectuée simultanément à la collision des jets de liquide ou à la suite de celle-ci.

2. Utilisation selon la revendication 1, **caractérisée en ce que** les diamètres de buses du réacteur à microjets se situent dans la plage 200 µm - 1 500 µm, et les pressions hydrauliques d'alimentation des buses du réacteur à microjets se situent dans la plage 50-800 bars, et de préférence la plage 100-350 bars, et l'angle de collision des jets est compris entre 135° et 180° et de manière plus préférée entre 170° et 180°, plusieurs jets de liquide (4) chargés en biomasse (1) entrant en collision à des vitesses comprises entre 100 m/s et 400 m/s, et de manière plus préférée entre 140 m/s et 254 m/s.

3. Utilisation selon l'une des revendications 1 à 2, **caractérisée en ce que** la collision des jets de liquide a lieu dans une enceinte remplie de gaz ou traversée par un gaz.

4. Utilisation selon l'une des revendications 1 à 3, **caractérisée en ce que** la destruction des cellules est initiée ou favorisée par l'ajout d'adjuvants dans la biomasse fluide (1), les adjuvants étant des enzymes, des sels, des solvants organiques, des acides ou des bases.

5. Utilisation selon l'une des revendications 1 à 4, **caractérisée en ce qu'**un gaz ou un gaz liquéfié (12) est introduit dans au moins un jet de liquide (4) avant la formation du jet.

6. Utilisation selon l'une des revendications 1 à 5, **caractérisée en ce que,** avant la formation du jet, au moins un jet de liquide (4) est porté à une température supérieure au point d'ébullition normal du liquide.

7. Utilisation selon l'une des revendications 1 à 6, **caractérisée en ce que** des réactifs nécessaires à la poursuite du traitement sont un composant d'au moins un des jets de liquide (4).

8. Utilisation selon la revendication 7, **caractérisée en ce que** les réactifs pour l'estérification d'acides gras d'une biomasse (1), libres ou liés dans des lipides, sont constitués d'un mélange d'un acide et d'un alcool.

9. Utilisation selon la revendication 7, **caractérisée en ce que** les réactifs pour l'estérification d'acides gras d'une biomasse (1) sont constitués d'un mélange d'un alcoolate et de l'alcool correspondant.

10. Utilisation selon la revendication 7, **caractérisée en ce que** le réactif de saponification d'acides gras d'une biomasse (1), libres ou liés dans des lipides, est constitué d'une base, de préférence une solution de soude ou une solution d'hydroxyde de potassium, ou d'un mélange d'une solution de soude et d'une solution d'hydroxyde de potassium.
